# EUROPEAN PATENT SPECIFICATION

(11) **EP 1 637 168 B1**
(45) Date of publication and mention of the grant of the patent: **21.01.2009**
(21) Application number: 04727918.7
(22) Date of filing: 16.04.2004
(51) Int. Cl.: A61L 2/18, A61L 9/14, A61L 101/22

(54) **USE OF A DIALKYLKETONE PEROXIDE AS BIOCIDAL, STERILIZING, ANTISEPTIC, DISINFECTING AND ANTI-PARASITIC AGENT**
VERWENDUNG EINES DIALKYLKETON-PEROXIDS ALS BIOZIDES, STERILISIERENDES, ANTISEPTISCHES, DESINFIZIERENDES UND ANTIPARASITISCHES MITTEL
UTILISATION D'UN PEROXYDE DE DIALKYLCETONE COMME AGENT BIOCIDE : STERILISANT, ANTISEPTIQUE, DESINFECTANT ET ANTIPARASITAIRE

(30) Priority: 16.04.2003 WO PCT/ES03/00178
(43) Date of publication of application: 22.03.2006
(73) Proprietor: Neochemical Desarrollos Avanzados, S.A., 28805 Alcala De Henares (ES)
(72) Inventor: CORONADO LUENGO, Alonso, E-28100 Alcobendas (ES); RANDEZ GARCÍA, Juan, José, E-28660 Boadilla Del Monte (ES)
(74) Representative: Carpintero Lopez, Francisco
(86) International application number: PCT/ES2004/000169
(87) International publication number: WO 2004/091671

(56) References cited:
- EP-A1- 0 775 439
- CH-A- 482 405
- JP-A- 3 074 469
- JP-A- 5 305 126
- JP-A- 6 321 711

## Description

### FIELD OF THE INVENTION

The invention is related to biocidal agents. In particular, the invention contemplates the use of a di(C1-C20) alkyl ketone peroxide as a sterilizing, antiseptic, disinfecting and anti-parasitic agent, and methods of sterilisation and disinfection that involves the application of compositions, which contain a di(C1-C20) alkyl ketone peroxide.

### BACKGROUND OF THE INVENTION

For several years the techniques of sterilisation by physical or chemical methods have become well known to experts in the field. Of the former, the application of heat or radiation and the use of filters should be highlighted. Of the latter, the use of chemical agents, either antiseptics or disinfectants, and/or sterilising agents, stand out.

The application of humid heat, for example using an autoclave, is a widely used method to destroy bacteria and spores in a short period of time, which does not leave any toxic residues, does not deteriorate the exposed material and is economical. However, it does present some inconveniences in as much as it does not permit solutions forming emulsions with water to be sterilised, it is corrosive to certain metallic instruments and damages instruments that are sensitive to heat, especially if they contain certain polymeric materials.

The application of dry heat, using a sterilising oven or through incineration, for example, is also used for disinfection, as it is not as corrosive to metallic instruments and it permits the sterilisation of substances in powder and nonaqueous material, as well as non-volatile viscous substances. However, this method requires a longer sterilisation time with respect to the application humid heat and furthermore, it still damages certain polymeric materials.

The application of ionising radiation is an economical method used to sterilise thermolabile materials. However, it cannot be used for culture medium or protein solutions because it produces alterations of their components. The application of ultraviolet radiation (weakly penetrating) is used to sterilise surfaces.

In respect to sterilisation by filtration, using filter membranes with a determined pore size is a method applied to oily emulsions or thermolabile solutions. However, the filters that are generally used in laboratories do not retain viruses or mycoplasms.

Among the chemical compounds employed, we find sterilising agents, disinfectants and antiseptics. The effectiveness of these agents depends on their concentration and pH, as well as on the time over which they are applied.

Among the antiseptic agents, the alcohols, iodine, ionic and amphoteric agents, organo-mercurial compounds and some colorants should be mentioned.

The alcohols do not destroy spores and have a slow germicide activity. Iodine, on the other hand is an oxidising agent that is used as a disinfectant of the skin despite the fact that it is an irritant and is only effective as a sporicide at high concentrations. The ionic and amphoteric agents are odourless antiseptics that do not stain, they are not corrosive to metals and are non-toxic, as well as being stable and cheap. However, they do not act as sporicides or tuberculicides, even at high concentrations. The organo-mercurial compounds, are highly toxic. Hydrogen peroxide is a mild antiseptic, with oxidising and free radical-producing capacity. It is used in gaseous form as a disinfectant of surfaces or for the decontamination of biological cabinets given that it does not posses the toxic and carcinogenic properties of ethylene oxide and formaldehyde. Finally, certain colorants are also used as antiseptics, such as acridine or derivatives of triphenylmethane.

Among the sterilising agents and/or disinfectants, chlorine and its derivatives, aldehydes, phenolic compounds, and the ethylene oxide can be mentioned.

Chlorine, hypochlorites and the chloramines are well known disinfectants in the state of the art. The chlorinated product most used en disinfection is sodium hypochlorite that is active against all bacteria, including spores, and is also effective at a wide range of temperatures. The bactericidal activity of sodium hypochlorite is due to the hypochlorous acid (HClO) and to the Cl₂ that is formed when the hypochlorite is diluted in water. Its activity is influenced by the presence of organic material since there may be substances capable of reacting with the chlorated compounds in the medium that diminish their effective concentration and that form organic compounds with carcinogenic properties. Furthermore, chlorine is an irritant and is corrosive to certain materials.

The aldehydes are alkylating agents that are used as disinfectants and sterilising agents, being sporicides. Glutaraldehyde is the only effective sterilising agent at cold temperatures, but it is fairly toxic and is classified as carcinogenic. Gaseous formaldehyde is used to decontaminate buildings, environments, etc., although it has the inconvenience of being a strong irritant and of losing activity in refrigerated environments.

The phenolic compounds are commonly used disinfectants. Phenol is not usually used as a disinfectant due to its unpleasant odour, because it is a strong irritant and because of the residue that remains following the treatment of surfaces. The most used phenol derivatives are hexachlorophene and the cresols that are very effective at low concentrations against vegetative forms of bacteria, although they are not effective against spores.

Similarly, ethylene oxide is a disinfectant agent used in gas sterilisation, generally in the pharmaceutical industry. It serves to sterilise thermolabile material but it is very dangerous due to the fact that it is highly inflammable and explosive, as well as being carcinogenic.

In the case of infections by prion pathogenic agents, the cleansing, disinfection and sterilisation of the floors and surfaces, or of surgical, hospital or laboratory material, must be performed by chemical decontamination in sodium hypochlorite (2% of free chlorine at room temperature during 1-2 hours) or in sodium hydroxide (1-2 N for 1-2 hours), followed by steam sterilisation in an pre-vacuum autoclave (a cycle at 134°C during 18 minutes, or six cycles of 3 minutes at 134 °C, for example).

Finally, in order to combat parasites and in particular, insects, mites and arachnids, the current state of the art compounds are of very diverse nature, among which those that should be highlighted: mineral arsenic, fluorinated, sulphur or selenium compounds; compounds based on mineral oils such as anthracene and petrol oils; vegetable compounds such as nicotine, pyrethrin or rotenone derivatives; or synthetic organic compounds such as organophosphorated, organochlorated or carbamate-type compounds. However, many of these have ceased to be used due to their potential toxic effects.

Hence, there is still a need in the state of the art to produce sterilizing, antiseptic, disinfecting and anti-parasitic agents that are not toxic and that as well as acting on a wide spectrum of organisms, particularly micro-organisms including spores, for a very wide range of applications of sterilisation, asepsia, disinfection and deparasitisation of all types of surfaces, objects, areas or organisms.

The dialkylketone peroxides have been known for some time in the state of the art. In particular, the methyl ethyl ketone peroxide is widely known for its industrial use in polymerisation for the curing of unsaturated polyester resins (see for example, the United States patent US 4,931,514, the United States patent application US 2002/0137972 or the international patent application WO 9518180).

Likewise, it is known that methyl ethyl ketone peroxide is used in primer compositions that are applied to substrates (metal, fibreglass, plastic, etc.) that will be painted (see for example, the European patent application EP 1241233 A).

On the other hand, a composition has been described as a fuel additive that includes a ketone peroxide, such as methyl ethyl ketone peroxide, acetone peroxide or a mixture of both (see the United States patent application US 4,482,352).

Similarly, it is known that compositions including dialkyl ketone peroxide can be used to conserve organic tissues. As such, in the European patent EP 0775439 compositions are described that contain (C1-C6) dialkyl ketone peroxides for the conservation, the anatomical preparation, or the partial regeneration of organic tissues of human or animal origin.

However, the use of dialkyl ketone peroxides *per se* as sterilizing, antiseptic, disinfecting or anti-parasitic agents has not been described or demonstrated.

Surprisingly, the present authors have discovered that a di(C1-C20) alkyl ketone peroxide can be used *per se* as sterilizing, antiseptic, disinfecting or anti-parasitic agents without any noxious effects, both from the toxicological and environmental point of view, something that is very uncommon among the disinfectants and parasiticides currently known and used in the state of the art, and something that is totally novel in the case of sterilising agents.

The absence of toxicity represents a top-level innovating characteristic, especially in the ambit of sterilisation, in that the few products that are actually commercialised have a very elevated level of toxicity. According to European law, and probably that of all developed countries, the existence of a less toxic product that fulfils the same functions obliges the user to employ it in substitution of the more toxic product. The legislation also foments and promotes the research and development of alternative technologies that reduce the level of danger in the workplace in all areas. It is for this reason that this product has an enormous inventive importance, constituting a clear advance in the existing technology at a worldwide level.

Therefore, the aim of the present invention is to provide the use of said di(C1-C20) alkyl ketone peroxide as non-toxic sterilizing, antiseptic, disinfecting or anti-parasitic agent with a very wide spectrum of activity in terms of the type of organisms on which they act (bacteria, virus, fungi, spores, mycobacteria, protozoa, algae, prions, parasites, etc.), and in as much as the type of applications in which they can be employed ( packing, medical and industrial instruments, healthcare environments and sanitary surfaces, premises, general surfaces, industrial installations, refrigeration towers, sanitary hot water circuits, purification of drinking water for human or animal consumption, etc.).

### OBJECT OF THE INVENTION

One object of the present invention is related to the use of a composition that comprises as the active ingredient a di (C1-C20) alkyl ketone peroxide of formula (I).

Another object of the present invention is related to a method of sterilisation, disinfection, asepsia or deparasitisation that involves the application of said composition.

### DETAILED DESCRIPTION OF THE INVENTION

The invention provides the use of a composition that comprises as the active ingredient a di (C1-C20) alkyl ketone peroxide of formula (I) as a sterilizing, antiseptic, disinfecting and anti-parasitic agent.

In the present invention, the term "sterilizing agent" refers to any chemical substance that eliminates all life forms, including spores. Likewise, the term antiseptics agent" refers to any chemical substance that prevents the growth or action of micro-organisms, be it through their destruction or by inhibiting their growth and activity, being a substance that can be applied to the body of a human or animal. Finally, the term "disinfecting agent" refers to any chemical substance that destroys the vegetative forms but not necessarily the resistant forms of pathogenic micro-organisms, being a substance that is applied to inanimate objects.

In the context of the invention, the term "anti-parasitic agent" refers to any physical or chemical agent to fight against parasites, eliminating them, repelling them or attracting them, including the products used directly or indirectly for human or veterinary hygiene. In particular, it refers to insecticides, miticides and arachnicides.

All these agents can be considered as biocides, given that they are active substances, or preparations that contain one or more active substances, destined to destroy, or repel, or inactivate the harmful or damaging organisms, to prevent their activity, or to fight against them by any means, through a biological or chemical action.

In one particular embodiment of the present invention, said di(C1-C20) alkyl ketone peroxide is employed as a bactericide, virucide, fungicide, sporicide, mycobactericide, protocide, algicide, prionicide, insecticide, arachnicide or miticide.

In the context of the invention the term di(C1-C20) alkyl ketone peroxide refers to a compound of the formula (I): 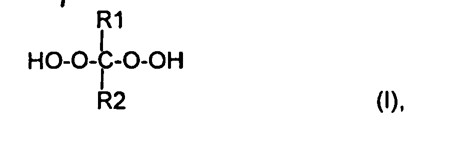 wherein R1 and R2 are the same or different, and they independently represent a (C1-C20) alkyl group, preferably a (C1-C6) alkyl group or a stereoisomer thereof, or a mixture thereof. Such alkyl groups can be linear or branched.

One of the preferred di(C1-C20) alkyl ketone peroxide that is encompassed within the present invention is methyl ethyl ketone peroxide.

In the case of ethyl methyl ketone peroxide, the following dimers are known: and their use comprised within the present invention.

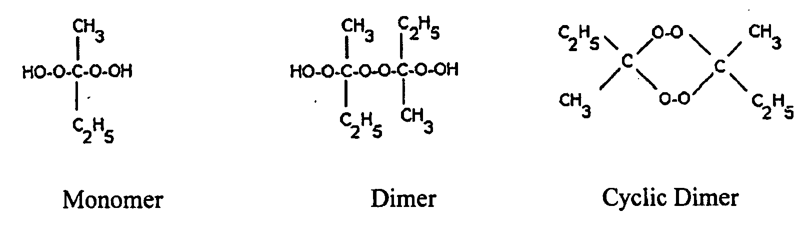

For the dimer, in particular, the D, L and meso- stereoisomers are also known.

In another specific embodiment of the present invention, the composition that comprises as the active ingredient a di (C1-C20) alkyl ketone peroxide of formula (I) is employed in very diverse applications in the areas including: packaging, wrappings, medical and industrial instruments, sanitary surfaces and healthcare environments, premises, surfaces in general which are not human or animal, industrial installations, refrigeration towers, air conditioning conduits, machinery and installations in food production, agriculture and fisheries installations, sanitary hot water circuits, purification of drinking water for human or animal consumption, or any other application in the industrial, domestic, environmental, agricultural, forestry, urban, pharmaceutical, civil, military, police enforcement, scientific, technological, spatial, geological, healthcare or health prevention areas for which the usefulness of the biocide properties of dialkyl ketone peroxide are demonstrated.

In human and animal therapy, the dialkyl ketone peroxide can be used as a bactericide, virucide, fungicide, sporicide, mycobactericide, protocide, algicide, prionicide or an anti-parasitic agent, by topical application to infected or infested skin, in distinct pharmaceutical formulations and forms, including those that are mentioned here: pomade, cream, lotion, solution, ointment, powder, solid bar, suspension, emulsion, nebuliser or spray.

Likewise, dialkyl ketone peroxide can also be used as a bactericide, virucide, fungicide, sporicide, mycobactericide, protocide, algicide, prionicide or an anti-parasitic agent, by enteral or parenteral, oral, rectal, vaginal, intramuscular, intradermic, intravenous or intra-arterial application, with the aim of combating infections by bacteria, mycobacteria, fungi, viruses, prions, protozoa, etc., in distinct pharmaceutical formulations and forms, including those that are mentioned here: pomade, cream, lotion, solution, ointment, powder, solid bar, suspension, emulsion, nebuliser, spray, syrup, enema, tablet, capsule, suppository, pessary, elixir or mouthwash.

In human hygiene, the dialkyl ketone peroxide is particularly useful in the formulation of products such as toothpastes and mouthwashes, for example, as an antiseptic at a concentration around 0.25% (v/v), with the additional advantage of its strong bleaching capacity of the dental enamel.

As mentioned above, the dialkyl ketone peroxide can be used as a disinfectant with high level-sterilising capacity to chemically sterilise surgical material that cannot be sterilised thermally, especially endoscopes, as well as the surfaces of operating theatres and clean rooms. It can also be used to disinfect materials that can be sterilised thermally, the use of such chemical sterilisation offering an alternative method of sterilisation.

On the other hand, di(C1-C20) alkyl ketone peroxide can be used as a disinfectant of organic residues, especially of the clinical or sanitary type, before their removal, in order to reduce their levels of infectious toxicity and in this way better comply with the laws concerning the Prevention of Risks in the Workplace and the Laws concerning Hazardous Waste Material.

In the same way, another use of the di(C1-C20) alkyl ketone peroxide is as an environmental disinfectant in order to disinfect all types of surfaces and non-surgical materials, such as laboratory, food industry, pharmaceutical industry, biotechnologic industry, etc.

The di(C1-C20) alkyl ketone peroxide has also been employed as an antiseptic disinfectant of healthy or damaged skin (with scars), or as liquid soap disinfectant to wash the hands hygienically with disinfection included. The preparation of this form is carried out by adding the product as an ingredient to a liquid soap.

Another of the uses of di(C1-C20) alkyl ketone peroxide is as a disinfectant of refrigeration towers against Legionella in refrigeration circuits. Its use consists in the addition of a determined quantity of the product, depending on the volume of water to be treated.

A further objective of the present invention is to provide the use of a composition comprising di(C1-C20) alkyl ketone peroxide as the active ingredient in a percentage by volume comprised between an effective amount and 5%.

In a preferred embodiment of the present invention, the use of a composition is provided as a bactericide, virucide, fungicide, sporicide, mycobactericide, protocide, algicide, prionicide, insecticide, arachnicide or miticide.

In a particular embodiment of the invention, the active ingredient is in a percentage by volume comprised between an effective amount and 0.3%.

In a preferred embodiment of the present invention, a composition is employed that comprises methyl ethyl ketone peroxide.

In a preferred embodiment of the present invention, a composition is used that comprises water, any adequate organic solvent, or an oil as an excipient. Among the adequate organic solvents, the alcohols are preferred, and in particular, an alcohol selected from hexylene glycol, polyethylene glycol, propylene glycol, glycerin-formal, diacetone alcohol, ethanol, n-propanol or isopropanol.

The preparation of said composition is carried out by conventional methods, by simply dissolving the di(C1-C20) alkyl ketone peroxide in the adequate solvent through mechanical agitation, preferably in a reactor for one hour.

The methyl ethyl ketone peroxide is commercially available through numerous suppliers at a worldwide level, given that it is a widely used product in industry. One of the commercial products available is Butanox M-50, whose declared concentration of methyl ethyl ketone peroxide is 33% (w/v), always expressed in an approximate form, the remaining 67% being the plasticizer (dimethyl phthalate). Likewise, any other commercial product can be used in which the concentration of peroxide generally, varies between 33 and 50% (w/v), the remaining percentage corresponding to a plasticizer such as dimethyl phthalate or isobutyl phthalate, or 2,2,4-trimethyl-1,3-pentane diol diisobutyrate, for example. The use of this latter plasticizer is particularly advantageous, since it avoids the possible release of phthalates.

Finally, the current invention provides a method of sterilisation, disinfection, asepsia or deparasitisation that comprises the application of composition that comprises as the active ingredient a di (C1-C20) alkyl ketone peroxide of formula (I) as previously described.

In a particular embodiment, the method of sterilisation, disinfection, asepsia or deparasitisation comprises the application of a composition that comprises as the active ingredient a di (C1-C20) alkyl ketone peroxide of formula (I) wherein the active ingredient is present in a percentage by volume comprised between an effective amount and 5%. In another particular embodiment the active ingredient is present in a percentage by volume comprised between an effective amount and 0.3%.

In another preferred embodiment, the method of sterilisation, disinfection, asepsia or deparasitisation comprises the application of composition comprising methyl ethyl ketone peroxide as active ingredient.

In another particular embodiment, the method of sterilisation, disinfection, asepsia or deparasitisation comprises the application of composition that comprises as the active ingredient a di (C1-C20) alkyl ketone peroxide of formula (I) that uses water, any adequate organic solvent, or an oil as an excipient. Among the adequate organic solvents, the alcohols are preferred and in particular, an alcohol selected from hexylene glycol, polyethylene glycol, propylene glycol, glycerin-formal, diacetone alcohol, ethanol, n-propanol or isopropanol.

The application of said composition is achieved through conventional methods. In the case of intense disinfection or sterilisation, the mode of employment is the manual immersion in a tank or a similar immersion performed automatically using washing/disinfection machines. In the remaining applications, said composition is employed by bringing the liquid product into contact with the surface to be disinfected as usual. Among the usual ways to achieve this, the following can be mentioned: nebulization with a nebuliser spray or a spray using propellant gases; dispensation by means of a mechanical device (such as the liquid soaps); pouring with or without dosification onto the hands, skin, an area, piping installation or vessel containing the liquid to be treated; the simple application by extending it with a brush, paintbrush, mop or cloth, or through a dropper, etc.

In the case of the application of the di(C1-C20) alkyl ketone peroxide to refrigeration towers in order to prevent or combat the Legionella bacteria, it is recommended to use a 5% dilution of the active ingredient in n-propanol and water, and to dilute it 1:50 such that the active ingredient is applied at the concentration of 0.1% (v/v). Likewise, in order to apply it in sanitary hot water systems, in the system the active ingredient should be diluted to a concentration of 0.1% (v/v). Similarly, for water circuits in the food industry, or for the purification of drinking water, the active ingredient should be diluted to 0.1% (v/v). Finally, for intense disinfection it is recommended that concentrations of 2% be used (v/v).

On the other hand, when referring to the applications for human hygiene (mouthwashes and toothpastes, for example), it is recommended that the di(C1-C20) alkyl ketone peroxide be used at a concentration in the order of 0.25% (v/v).

The following examples illustrate the invention.

### Example of a topical formulation:

### Product 1- NEOSTEX

### Antiseptic for the washing of hands

### Complete Quantitative Composition

| | | CAS N. | g/100 ml |
|---|---|---|---|
| Active ingredient: | | | |
| | Methyl-ethyl-ketone peroxide | 1338-23-4 | 0.25 |
| Other components: | | | |
| | N-propanol | 71-23-8 | 70 |
| | Water | 7732-18-5 | 20 |
| | Glycerin | 56-81-5 | 5 |
| | Isopropanol | 67-63-0 | 4.5 |
| | Menthol | 89-78-1 | 0.25 |
| | Dimethyl Phthalate | 131-11-3 | 0.50 |

### Product 2- NEOSTEX PLUS

### Antiseptic for the washing of hands

### Complete Quantitative Composition

| Active ingredient: | | CAS N. | g/100 ml |
|---|---|---|---|
| | Methyl-ethyl-ketone peroxide | 1338-23-4 | 0.25 |
| Other components: | | | |
| | Isopropanol | 67-63-0 | 70 |
| | Water | 7732-18-5 | 20 |
| | Glycerin | 56-81-5 | 5 |
| | N-propanol | 71-23-8 | 4.5 |
| | Menthol | 89-78-1 | 0.25 |
| | Dimethyl Phthalate | 131-11-3 | 0.50 |

### Examples of activity

### EXAMPLE 1

### Bactericidal Activity

Three solutions of methyl ethyl ketone peroxide were prepared at 0.06%, 0.125% and 0.25% (v/v) by diluting Butanox M-50 (approximately 33% methyl ethyl ketone peroxide; w/v) in sterile hard water (300 mg/l of CaCO₃). A neutralising solution of thioglycolate was added at 0.5%, in this way producing three clear and colourless solutions.

Each of the aforementioned solutions was brought into contact with distinct bacterial strains for 5, 15 and 30 minutes at a temperature of 20°C (Pseudomonas aeruginosa ATCC 15442, Escherichia coli ATCC 10536, Staphylococcus aureus ATCC 6538, Enterococcus hirae ATCC 8043 and Legionella pneumophila ATCC 33152) and incubated at 20°C.

The data from the validation assays are shown in Table I and the results of the bactericidal activity of each one of the three methyl ethyl peroxide solutions are shown in Table 1.

**TABLE I -Validation Assay**

| Test organism | Bacterial suspension | Experimental conditions with interfering substance | Toxicity control of the neutralising agent | Control of the method of dilution-neutralisation with the interfering substance |
|---|---|---|---|---|
| Pseudomonas aeruginosa | **Vc** | **Vc** | **Vc** | **Vc** |
| ATCC 15442 | 132; 142 | 160; 145 | 121; 124 | 120; 110 |
| | **Nv** 137 | **A** 152 | **B** 122 | **C** 115 |
| Escherichia coli | **Vc** | **Vc** | **Vc** | **Vc** |
| ATCC 10536 | 175; 168 | 170; 160 | 160; 157 | 140; 131 |
| | **Nv** 171 | **A** 165 | **B** 158 | **C** 135 |
| Staphylococcus aureus | **Vc** | **Vc** | **Vc** | **Vc** |
| ATCC 6538 | 123; 126 | 102; 132 | 115; 107 | 100; 90 |
| | **Nv** 124 | **A** 117 | **B** 111 | **C** 95 |
| Enterococcus hirae | **Vc** | **Vc** | **Vc** | **Vc** |
| ATCC 8043 | 118; 113 | 116; 131 | 120; 118 | 115; 100 |
| | **Nv** 115 | **A** 123 | **B** 119 | **C** 107 |
| Legionella pneumophila | **Vc** | **Vc** | **Vc** | **Vc** |
| ATCC 33152 | 135; 160 | 127; 140 | 120; 162 | 128; 180 |
| | **Nv** 147 | **A** 133 | **B** 141 | **C**154 |

| | | | | |
|---|---|---|---|---|
| **Vc:** viable count; **Nv:** number of CFU/ml in the bacterial suspension; **A:** number of CFU/ml in the validation assay of the experimental conditions used in this method (with interfering substances); **B:** number of CFU/ml in the validation assay of the toxicity of the neutralising agent; **C:** number of CFU/ml in the validation assay of the method of dilution-neutralisation. | | | | |

**TABLE 1- Bactericidal Activity**

| Test organism | Bacterial suspension in the assay | | t = 5 min | | | | t = 15 min | | | t = 30 min | | |
|---|---|---|---|---|---|---|---|---|---|---|---|---|
| | | | | 0.06% | 0.125% | 0.25% | 0.06% | 0.125% | 0.25% | 0.06% | 0.125% | 0.25% |
| | | | | (v/v) | (v/v) | (v/v) | (v/v) | (v/v) | (v/v) | (v/v) | (v/v) | (v/v) |
| Pseudomonas | **Vc** | 10⁻⁶: 109;134 | **Vc** | 2; 2 | 0; 0 | 0; 0 | 0; 0 | 0; 0 | 0; 0 | or 0 | 0; 0 | 0; 0 |
| aeruginosa | | 10⁻⁷ : 20; 14 | **Nv** | 0.2 x 10² | 0 | 0 | 0 | 0 | 0 | 0 | 0 | 0 |
| ATCC 15442 | **N** | 4.8 x 10⁸ | **R** | 24 x 10⁵ | ≥ 10⁶ | ≥ 10⁶ | ≥ 10⁶ | ≥ 10⁶ | ≥ 10⁶ | ≥ 10⁶ | ≥ 10⁶ | ≥ 10⁶ |
| Escherichia coli | **Vc** | 10⁻⁶:178; 175 | **Vc** | 63; 69 | 0; 0 | 0; 0 | 0; 0 | 0; 0 | 0; 0 | 0; 0 | 0; 0 | 0; 0 |
| ATCC 10536 | | 10⁻⁷ : 16; 26 | **Na** | 6.6 x 10² | 0 | 0 | 0 | 0 | 0 | 0 | 0 | 0 |
| | **N** | 2.1 x 10⁸ | **R** | 0.3 x 10⁵ | ≥10⁶ | ≥ 10⁶ | ≥ 10⁶ | ≥ 10⁶ | ≥ 10⁶ | ≥ 10⁶ | ≥ 10⁶ | ≥ 10⁶ |
| Staphylococcus | **Vc** | 10⁻⁶: 160;180 | **Vc** | 72; 164 | 35; 18 | 0; 0 | 115; 120 | 0; 2 | 0; 0 | 2; 1 | 0; 0 | 0; 0 |
| aureus | | 10⁻⁷: 12; 19 | **Na** | 11 x 10² | 2.6 x 10² | 0 | 11.7 x 102 | 0.1 x 10² | 0 | 0.15 x 10² | 0 | 0 |
| ATCC 6538 | **N** | 1.5 x 10⁸ | **R** | 0.1 x 10⁵ | 0.5 x 10⁵ | ≥ 10⁶ | 0.08 x 10⁵ | 15 x 10⁵ | ≥ 10⁶ | 10 x 10⁵ | ≥ 10⁶ | ≥ 10⁶ |
| Enterococcus | **Vc** | 10⁻⁶: 240;255 | **Vc** | 245; 235 | 176; 180 | 0; 2 | 240; 256 | 70; 69 | 0; 0 | 208; 202 | 0; 0 | 0; 0 |
| hirae | | 10⁻⁷: 28; 20 | **Na** | 24 x 10² | 17.8 x 10² | 0.1 x 10² | 24.8 x 10² | 6.9 x 10² | 0 | 20.5 x 10² | 0 | 0 |
| ATCC 8043 | **N** | 2.4 x 10⁸ | **R** | 0.1 x 10⁵ | 0.1 x 10⁵ | 24 x 10⁵ | 0.09 x 10⁵ | 0.3 x 10⁵ | ≥ 10⁶ | 0.1 x 10⁵ | ≥ 10⁶ | ≥ 10⁶ |

| | | | | | | | | | | | | |
|---|---|---|---|---|---|---|---|---|---|---|---|---|
| Vc: viable count; N: number of CFU/ml in the bacterial suspension in the assay; Na: number of CFU/ml in the assay mixture (< 1.5 x 10² or > 3 x 10³ CFU/ml); R: Reduction of viability (for a bactericidal effect to exist, **R** must be > **10⁵**) | | | | | | | | | | | | |

### Conclusion

The assays performed with the product dealt with in this invention, according to the norm EN 1276 (1997) and at dilutions of 0.06%, 0.125% and 0.25% (v/v) in hard water, demonstrate that said product possesses a bactericidal activity against: Pseudomonas aeruginosa within 5, 15 and 30 minutes at the concentration of 0.06%; against Escherichia coli within 5 minutes at a concentration of 0.125% and within 15 and 30 minutes at a concentration of 0.06%; against Staphylococcus aureus within 5 and 15 minutes at 0.125% and within 30 minutes at 0.06%; against Enterococcus hirae within 5 and 15 minutes at 0.25% and within 30 minutes at 0.125%; and against Legionella pneumophila within 5 minutes at 0.125% and within 15 and 30 minutes at a concentration of 0.06%.

### EXAMPLE 2

### Fungicidal Activity

Three solutions of methyl ethyl ketone peroxide were prepared at 0.06%, 0.125% and 0.25% (v/v) by diluting Butanox M-50 (approximately 33% methyl ethyl ketone peroxide; w/v) in a sodium chloride/tryptone solution. A solution of thioglycolate (0.5%) was added as a neutralising agent.

Each of the solutions was brought into contact with the fungal strains Candida albicans ATCC 10321 and Aspergillus niger ATCC 16404, for 5, 15 and 30 minutes at a temperature of 20°C and incubated at 30 °C.

The data from the validation assays are presented in Table II and the results of the fungicidal activity of each of the three solutions of methyl ethyl ketone peroxide are laid out in Table 2.

**TABLE II -Validation Assays**

| Test organism | Fungal suspension | Experimental conditions | Toxicity of the neutralising agent | Validation assay of the method of neutralisation-dilution |
|---|---|---|---|---|
| Aspergillus niger ATCC 16404 | **Vc** 20; 70 | **Vc** 70; 20 | **Vc** 60; 50 | **Vc** 130; 90 |
| | **Nv** 4.5 x 10² | **A** 4.5x10¹ | **B** 5.5 x 10¹ | **C** 1.1 x 10² |
| Candida albicans ATCC 10321 | **Vc** 140; 120 | **Vc** 100; 120 | **Vc** 160; 110 | **Vc** 110; 130 |
| | **Nv** 1.3x10³ | **A** 1.1 x 10² | **B** 1.3 x 10² | **C** 1.2 x 10² |

| | | | | |
|---|---|---|---|---|
| **Vc:** viable count; **Nv:** number of CFU/ml in the fungal suspension; **A:** number of CFU/ml in the validation assay of the experimental conditions (with interfering substances); **B:** number of CFU/ml in the validation assay of the toxicity of the neutralising agent; **C:** number of CFU/ml in the validation assay of the method of dilution-neutralisation. | | | | |

**TABLE 2- Fungicidal Activity**

| Test organism | Fungal suspension in the assay | | | t = 5 min | | | t = 15 min | | | t = 30 min | | |
|---|---|---|---|---|---|---|---|---|---|---|---|---|
| | | | | 0.06% | 0.125% | 0.25% | 0.06% | 0.125% | 0.25% | 0.06% | 0.125% | 0.25% |
| | | | | (v/v) | (v/v) | (v/v) | (v/v) | (v/v) | (v/v) | (v/v) | (v/v) | (v/v) |
| Aspergillus niger | **Vc** | 10⁻⁵ 50; 57 | **Vc** | 4; 4 | 2; 1 | 0; 0 | 2; 3 | 2; 1 | 0; 0 | 4; 8 | 1; 1 | 0; 0 |
| ATCC 16404 | | 10⁻⁶ 5; 6 | **Na** | 0.4 x 10² | 0.1 x 10² | 0 | 0.2 x 10² | 0.1 x 10² | 0 | 0.6 x 10² | 0.1 x 10² | 0 |
| | **N** | 5.5 x 10⁶ | **R** | 1.3 x 10⁴ | 3.6 x 10⁴ | ≥ 10⁵ | 1.3 x 10⁴ | 5.5 x 10⁴ | ≥ 10⁵ | 1.3 x 10⁴ | 5.5 x 10⁴ | ≥ 10⁵ |
| Candida albicans | **Vc** | 10⁻⁵ 150. 170 | **Vc** | 5; 7 | 2; 0 | 0; 0 | 0; 0 | 0; 0 | 0; 0 | 0; 0 | 0; 0 | 0; 0 |
| ATCC 10321 | | 10⁻⁶ 16; 15 | **Na** | 0.6 x 10² | 0.1 x 10² | 0 | 0 | 0 | 0 | 0 | 0 | 0 |
| | **N** | 1.5 x 10⁷ | **R** | 2.5 x 10⁴ | 15 x 10⁴ | ≥ 10⁵ | ≥ 10⁵ | ≥ 10⁵ | ≥ 10⁵ | 0.9x10⁴ | ≥ 10⁵ | ≥ 10⁵ |

| | | | | | | | | | | | | |
|---|---|---|---|---|---|---|---|---|---|---|---|---|
| **Vc:** viable count; **N:** number of CFU/ml of the fungal suspension under assay; **Na:** number of CFU/ml in the assay mixture; **R:** reduction of viability | | | | | | | | | | | | |

### Conclusion

In accordance with the norm EN 1275 (October 1997), the product that is the subject of this invention possess a fungicidal activity towards the strains mentioned: Candida albicans ATCC 10321 and Aspergillus niger ATCC 16404.

### EXAMPLE 3

### Sporicidal Activity

Three solutions of methyl ethyl ketone peroxide were prepared at 15%, 20% and 25% (v/v) by diluting Butanox M-50 (approximately 33% methyl ethyl ketone peroxide; w/v) in a sodium chloride/tryptone solution. Each of the solutions was brought into contact with a suspension of Bacillus subtilis spores ATCC 19659, for 5, 15 and 30 minutes at a temperature of 20°C, incubated at 35 °C, and placed on a carrier disc in the presence of mucin and bovine serum albumin.
Method used: A carrier disc according to the norm ASTM E-2197-02. Standard Quantitative Disk carrier test method for determining the bactericidal, virucidal, fungicidal, mycobactericidal and sporicidal activities of liquid chemical germicides. ASTM International, Pa, USA.

| Suspension of Bacillus subtilis spores | |
|---|---|
| ATCC 19659 of the assay | 8.6 x 10² CFU/ml |
| Control of the suspension in the presence of mucin | 8.1 x 10² CFU/ml |
| Control of the suspension in the presence of bovine serum albumin | 7.2 x 10² CFU/ml |

The mean number of colony forming units per ml (CFU/ml) was determined after the exposure of 10 test carrier disks to each concentration of the methyl ethyl ketone peroxide indicated for the time specified. The results of the sporicidal activity of each one of the three solutions of methyl ethyl ketone peroxide are shown in Table 3.

**TABLE 3 - Sporicidal Activity**

| Concentration of peroxide (%) | Exposure time | | |
|---|---|---|---|
| | 5 min | 15 min | 30 min |
| 15% | 7.2 x 10² | 4.3 x 10² | 1.8 x 10² |
| 20% | 7.6 x 10² | 0.5 x 10² | 0 |
| 25% | 5.0 x 10² | 0.3 x 10² | 0 |

### Conclusion

The product that is dealt with in this invention, when used at the concentration of 20% and 25% during 30 minutes, possesses absolute sporicidal activity under the specific conditions of this assay. When used during 15 at a concentration of 20% and 25%, the sporicidal activity is not absolute although it significantly reduces the number of viable spores.

### EXAMPLE 4

### Virucidal Activity

A solution of methyl ethyl ketone peroxide was prepared at 0.25% (v/v) by diluting Butanox M-50 (approximately 33% methyl ethyl ketone peroxide; w/v) in cell culture medium. This solution was brought into contact with a suspension of poliovirus type 1 ATCC VR-192 for 15 minutes at a temperature of 20°C; (incubation temperature: 35 °C).
The Method utilised: ASTM E-1053-97, is a Standard Test Method for Efficacy of Virucidal Agents Intended for Inanimate Environmental Surfaces. ASTM International, Pa, USA.
- Suspension of Poliovirus type 1 ATCC VR-192 assayed 1 x10⁷ TCID₅₀
- Cell line used Vero cells.
- Control of the poliovirus type 1 suspension used under the assay conditions without exposure to disinfectant, with titre in base 10 in order to calculate the TCID₅₀ units. Four replicates per dilution.
- Control of cytotoxicity of the disinfectant: observations of the effect on each cell monolayer inoculated with disinfectant at dilutions in base 10 and intermediates in base 2. Four replicates per dilution.
- Control of the cell monolayer, four replicates over 4 days of observation.
- Method of disinfectant neutralisation: dilution in cell culture medium until it is no longer cytotoxic: 1:7,000 with four replicates.

The mean number of infective units per ml (TCID₅₀) for a cell culture was determined after exposing the 10 monolayers to each of the concentrations of the product of the invention for the time indicated. The results of the virucidal activity of the solution of methyl ethyl ketone peroxide are shown in Table 4.

**TABLE 4- Virucidal Activity**

| **PRODUCT CONCENTRATION** (%) | Exposure time | | |
|---|---|---|---|
| | | 15 min | |
| 0.25% in cell culture medium | | Absence of CPE Reduction (log₁₀): ≥ 5 | |
| **CONTROLS** | | | |
| Control viral suspension without disinfectant | | 1x10⁴ TClD₅₀ | |
| Control of cytotoxicity | | Absence of CTX | |
| Control of cells | | Normal cells | |
| Control of the neutralisation of the disinfectant | | Normal cells | |

| | | | |
|---|---|---|---|
| CPE: cytopathic effect; CTX: cytotoxicity. | | | |

### Conclusion

The product dealt with in this invention possesses an absolute virucidal effect at a concentration of 0.25% within 15 minutes, with a reduction > 1 x 10⁴ TCID₅₀ against Poliovirus type 1 under the conditions indicated.

### EXAMPLE 5

### Mycobactericidal Activity

Three solutions of methyl ethyl ketone peroxide were prepared at 1 %, 2% and 4% (v/v) by diluting Butanox M-50 (approximately 33% methyl ethyl ketone peroxide; w/v) in a solution of sodium chloride-tryptone. Each of these solutions was brought into contact with a suspension of Mycobacterium terrae ATCC 15755 for 5, 15 and 30 minutes at a temperature of 20°C, incubated at 35 °C and then placed on a carrier disk in the presence of mucin and bovine serum albumin.

The method used: ASTM E-2197-02. Standard Quantitative Disk carrier test method for determining the bactericidal, virucidal, fungicidal, mycobactericidal and sporicidal activities of liquid chemical germicides. ASTM International, Pa, USA.
- Suspension of Mycobacterium terrae ATCC 15755 in the assay ........2,100 CFU/ml
- Control of the suspension in the presence of mucin ..........................1,700 CFU/ml
- Control of the suspension in the presence of bovine serum albumin 1,850 CFU/ml

The mean number of colony forming units per ml (CFU/ml) recovered was determined after the exposure of the 10 test carrier disks to each one of the three test solutions of methyl ethyl ketone peroxide indicated during the time stipulated.

**TABLE 5- Mycobactericidal Effect**

| Concentration of peroxide (%) | Exposure time (minutes) | | |
|---|---|---|---|
| | 5 min | 15 min | 30 min |
| 0.5% | 175 | 100 | 90 |
| 1% | 160 | 0 | 0 |
| 2% | 0 | 0 | 0 |
| 4% | 0 | 0 | 0 |

### Conclusion

The product dealt with in this invention possesses a mycobactericidal effect at a concentration of 1% within 15 and 30 minutes, and at the concentrations of 2% and 4%, the mycobactericidal effect can be seen at 5, 15 and 30 minutes.

## Claims

1. Use of a composition that comprises as the active ingredient a di(C1-C20)alkyl ketone peroxide of formula (I): 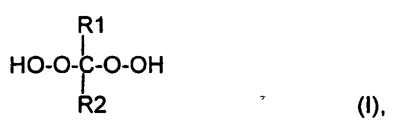 or a stereoisomer thereof, or a mixture thereof, in a percentage by volume comprised between an effective amount and 5%, wherein R1 and R2 represent independently an alkyl group linear or branched, as a biocide, sterilizing, antiseptic, disinfecting or anti-parasitic agent.

2. The use of a composition according to claim 1, wherein the active ingredient is present in a percentage by volume comprised between an effective amount and 0.3%.

3. The use of a composition according to claim 1 or 2, wherein the active ingredient is a di(C1-C6)alkyl ketone peroxide.

4. The use according to anyone of previous claims, **characterised in that** said composition comprises methyl ethyl ketone peroxide.

5. The use according to anyone of previous claims, **characterised in that** said composition comprises water, or an adequate organic solvent or an oil as an excipient.

6. The use according to claim 5, **characterised in that** the organic solvent is an alcohol.

7. The use according to claim 6, **characterised in that** the alcohol is selected from: hexylene glycol, polyethylene glycol 200, propylene glycol and glycerin-formal, diacetone alcohol, ethanol, n-propanol or isopropanol

8. The use according to anyone of claims 1-7, as a bactericide.

9. The use according to anyone of claims 1-7, as a virucide.

10. The use according to anyone of claims 1-7, as a fungicide.

11. The use according to anyone of claims 1-7, as a sporicide.

12. The use according to anyone of claims 1-7, as a mycobactericide.

13. The use according to anyone of claims 1-7, as a protocide.

14. The use according to anyone of claims 1-7, as an algicide.

15. The use according to anyone of claims 1-7, as a prionicide.

16. The use according to anyone of claims 1-7, as an insecticide.

17. The use according to anyone of claims 1-7, as an arachnicide.

18. The use according to anyone of claims 1-7, as a miticide.

19. The use according to the previous claims, in packing, wrapping, medical and industrial instruments, on sanitary surfaces and healthcare environments, premises, industrial installations, refrigeration towers, air conditioning conduits, machinery and installations in the food industry, agriculture and fisheries installations, sanitary hot water circuits, purification of drinking water for human or animal consumption, or any other industrial, domestic, environmental, agricultural, forestry, urban, civil, military, police purposes, scientific, technological, spatial, geological application.

20. Use of a composition that comprises as the active ingredient a di(C1-C20)alkyl ketone peroxide of formula (I): 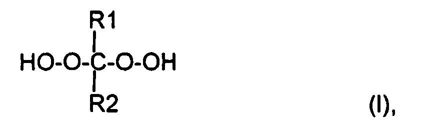 or a stereoisomer thereof, or a mixture thereof, in a percentage by volume comprised between an effective amount and 5%, wherein R1 and R2 represent independently an alkyl group linear or branched, in the elaboration of a medicament for the treatment or profilaxis of an infection.

## Patentansprüche

1. Verwendung einer Zusammensetzung, die als wirksamen Bestandteil ein Di (C₁- bis C₂₀)Alkylketonperoxid mit der Formel (I) : 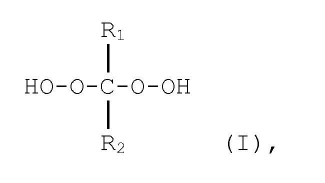 ein Stereoisomer oder ein Gemisch davon mit einem Volumenprozentsatz von einem wirksamen Anteil bis zu 5 % umfasst, wobei R₁ und R₂ unabhängig voneinander eine geradkettige oder verzweigte Alkylgruppe bedeuten, als biozides, sterilisierendes, antiseptisches, desinfizierendes oder Parasiten bekämpfendes Mittel.

2. Verwendung einer Zusammensetzung nach Anspruch 1, wobei der wirksame Bestandteil mit einem Volumenprozentsatz von einem wirksamen Anteil bis zu 0,3 % enthalten ist.

3. Verwendung einer Zusammensetzung nach Anspruch 1 oder 2, wobei der wirksame Bestandteil ein Di(C₁- bis C₂₀)Alkylketonperoxid ist.

4. Verwendung nach einem der vorhergehenden Ansprüche, **dadurch gekennzeichnet, dass** die Zusammensetzung Methylethylketonperoxid umfasst.

5. Verwendung nach einem der vorhergehenden Ansprüche, **dadurch gekennzeichnet, dass** die Zusammensetzung Wasser, ein geeignetes organisches Lösungsmittel oder ein Öl als Grundlage enthält.

6. Verwendung nach Anspruch 5, **dadurch gekennzeichnet, dass** das organische Lösungsmittel ein Alkohol ist.

7. Verwendung nach Anspruch 6, **dadurch gekennzeichnet, dass** der Alkohol aus Hexylenglykol, Polyethylenglykol 200, Propylenglykol und Glycerin-Formal, Diacetonalkohol, Ethanol, n-Propanol oder Isopropanol ausgewählt ist.

8. Verwendung nach einem der Ansprüche 1 bis 7 als Bakterizid.

9. Verwendung nach einem der Ansprüche 1 bis 7 als Viruzid.

10. Verwendung nach einem der Ansprüche 1 bis 7 als Fungizid.

11. Verwendung nach einem der Ansprüche 1 bis 7 als Sporizid.

12. Verwendung nach einem der Ansprüche 1 bis 7 als Mykobakterizid.

13. Verwendung nach einem der Ansprüche 1 bis 7 als Protozid.

14. Verwendung nach einem der Ansprüche 1 bis 7 als Algizid.

15. Verwendung nach einem der Ansprüche 1 bis 7 als Prionizid.

16. Verwendung nach einem der Ansprüche 1 bis 7 als Insektizid.

17. Verwendung nach einem der Ansprüche 1 bis 7 als Arachnizid.

18. Verwendung nach einem der Ansprüche 1 bis 7 als Akarizid.

19. Verwendung nach den vorhergehenden Ansprüchen für das Verpacken, Umhüllen, medizinische und technische Instrumente, auf sanitären Oberflächen und Umgebungen der Gesundheitspflege, für Grundstücke, Industrieanlagen, Kühltürme, Klimaanlagen, Maschinen und Anlagen in der Lebensmittelindustrie, landwirtschaftliche und Fischereianlagen, sanitäre Warmwasserleitungen, Reinigung von Trinkwasser für Mensch bzw. Tier oder einen beliebigen anderen industriellen, häuslichen, umwelttechnischen, landwirtschaftlichen, forstwirtschaftlichen, städtischen, zivilen, militärischen, polizeilichen, wissenschaftlichen, technologischen, raumfahrttechnischen und geologischen Zweck.

20. Verwendung einer Zusammensetzung, die als wirksamen Bestandteil ein Di (C₁- bis C₂₀)Alkylketonperoxid mit der Formel (I): 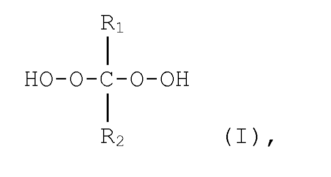 ein Stereoisomer oder ein Gemisch davon mit einem Volumenprozentsatz von einem wirksamen Anteil bis zu 5 % umfasst, wobei R₁ und R₂ unabhängig voneinander eine geradkettige
oder verzweigte Alkylgruppe bedeuten, zur Herstellung eines Arzneimittels für die Behandlung oder Prophylaxe einer Infektion.

## Revendications

1. Utilisation d'une composition qui comprend comme ingrédient actif un peroxyde de dialkylcétone (C1-C20) de la formule (I) : 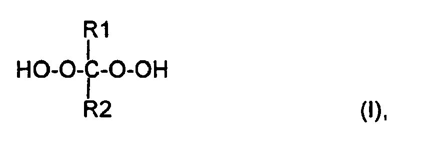 ou un stéréoisomère de celui-ci, ou un mélange de celui-ci, en un pourcentage en volume compris entre une quantité efficace et 5%, où R1 et R2 représentent indépendamment un groupe alkyle linéaire ou branché, comme un agent biocide, stérilisant, antiseptique, désinfectant ou anti-parasitaire.

2. Utilisation d'une composition selon la revendication 1, où l'ingrédient actif est présent en un pourcentage en volume compris entre une quantité efficace et 0,3%.

3. Utilisation d'une composition selon la revendication 1 ou 2, où l'ingrédient actif est un peroxyde de dialkylcétone (C1-C6).

4. Utilisation selon l'une quelconque des revendications précédentes, **caractérisée en ce que** ladite composition comprend un peroxyde méthyl éthyl cétone.

5. Utilisation selon l'une quelconque des revendications précédentes, **caractérisée en ce que** ladite composition comprend de l'eau, ou un solvant organique adéquat ou une huile comme excipient.

6. Utilisation selon la revendication 5, **caractérisée en ce que** le solvant organique est un alcool.

7. Utilisation selon la revendication 6, **caractérisée en ce que** l'alcool est sélectionné parmi: hexylène glycol, polyéthylène glycol 200, propylène glycol et glycérinformal, diacétone alcool, éthanol, n-propanol ou isopropanol.

8. Utilisation selon l'une quelconque des revendications 1 à 7, comme un bactéricide.

9. Utilisation selon l'une quelconque des revendications 1 à 7, comme un virucide.

10. Utilisation selon l'une quelconque des revendications 1 à 7, comme un fongicide.

11. Utilisation selon l'une quelconque des revendications 1 à 7, comme un sporicide.

12. Utilisation selon l'une quelconque des revendications 1 à 7, comme un mycobactéricide.

13. Utilisation selon l'une quelconque des revendications 1 à 7, comme un protocide.

14. Utilisation selon l'une quelconque des revendications 1 à 7, comme un algicide.

15. Utilisation selon l'une quelconque des revendications 1 à 7, comme un prionicide.

16. Utilisation selon l'une quelconque des revendications 1 à 7, comme un insecticide.

17. Utilisation selon l'une quelconque des revendications 1 à 7, comme un arachnicide.

18. Utilisation selon l'une quelconque des revendications 1 à 7, comme un miticide.

19. Utilisation selon les revendications précédentes, pour l'emballage, l'enroulement, des instruments médicaux et industriels, sur des surfaces sanitaires et environnements de soins de santé, locaux, installations industrielles, tours de réfrigération, conduites de conditionnement d'air, machineries et installations dans l'industrie alimentaire, l'agriculture et les installations de pêche, circuits d'eau chaude sanitaire, purification de l'eau potable pour la consommation par l'homme ou l'animal ou toute autre application industrielle, domestique, environnementale, agricole, forestière, urbaine, civile, militaire, pour la police, scientifique, technologique, spatiale, géologique.

20. Utilisation d'une composition qui comprend comme ingrédient actif un peroxyde de dialkylcétone (C1-C20) de la formule (I) : 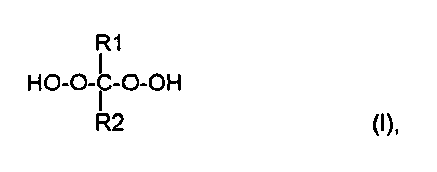 ou un stéréoisomère de celui-ci, ou un mélange de celui-ci, en un pourcentage en volume compris entre une quantité efficace et 5%, où R1 et R2 représentent indépendamment un groupe alkyle linéaire ou branché, dans l'élaboration d'un médicament pour le traitement ou la prophylaxie d'une infection.
